# EUROPÄISCHE PATENTSCHRIFT

(11) **EP 4 113 071 B1**
(45) Veröffentlichungstag und Bekanntmachung des Hinweises auf die Patenterteilung: **25.09.2024**
(21) Anmeldenummer: 22178939.9
(22) Anmeldetag: 14.06.2022
(51) Int. Cl.: G01F 13/00, G01F 22/00, B05B 15/25, B05B 15/58, B05C 11/10, G01N 21/64, B05C 5/02

(54) **AUSBRING-VORRICHTUNG MIT EINEM GAS-SENSOR SOWIE VERFAHREN FÜR IHREN BETRIEB**
SPREADER DEVICE WITH A GAS SENSOR AND METHOD FOR ITS OPERATION
DISPOSITIF D'ÉPANDAGE POURVU DE CAPTEUR DE GAZ ET SON PROCÉDÉ DE FONCTIONNEMENT

(30) Priorität: 02.07.2021 DE 102021117163
(43) Veröffentlichungstag der Anmeldung: 04.01.2023
(73) Patentinhaber: SCHEUGENPFLUG GmbH, 93333 Neustadt/Donau (DE)
(72) Erfinder: Piller, Sebastian, 90489 Nürnberg (DE); Dollinger, Stefan, 91334 Hemhofen (DE)
(74) Vertreter: Weickmann & Weickmann PartmbB

(56) Entgegenhaltungen:
- WO-A1-2019/037970
- DE-A1- 102005 048 969
- DE-A1- 102016 123 586
- DE-A1- 102017 126 732
- DE-A1- 19 651 423

## Beschreibung

### I. Anwendungsgebiet

Die Erfindung betrifft eine Ausbring-Vorrichtung für flüssiges und viskoses Material wie etwa Kleber, Gießharze oder Vergussmassen, in deren Aufbereitungs-Einheit dieses Material nicht nur bevorratet, sondern durch Rühren und Zirkulieren auch am Entmischen gehindert wird und vor allem auch entgast wird, da für die weitere Verwendung in einem Verbraucher, meist einer Ausbring-Einheit, Gas, insbesondere Luft, insbesondere sichtbare Lufteinschlüsse oder auch gelöste Luft, in dem Material äußerst nachteilig sind.

In diesem Zusammenhang ist strittig, was unter gelöster Luft zu verstehen ist. Die Mehrheit der Meinungen definiert gelöste Luft als eine geringe Ansammlung von Luft in Form von sehr kleinen, nicht mehr sichtbaren Luftblasen, im Material, nicht zu verwechseln mit Ionen aus den chemischen Elementen, aus denen Luft besteht.

### II. Technischer Hintergrund

Eine solche Aufbereitungs-Einheit umfasst zunächst den Aufbereitungs-Behälter, der in der Aufsicht betrachtet meist einen runden Innen-Querschnitt besitzt, damit bei Anordnen eines um eine aufrechte Achse drehenden Rührers darin dessen Rührflügel meist entlang des Umfanges nahe an den Innenflächen der Wandung und/oder dem Boden des Vorratsbehälters entlang bewegt werden können.

Für das aufzubereitende Material gibt es im Aufbereitungs-Behälter eine ortsfeste, in aller Regel dezentrale, Zufluss-Öffnung in dessen oberen Bereich und eine Abfluss-Öffnung im unteren Bereich, die meist gekoppelt ist mit einer oder zwei Förderpumpen, um das Material über eine Versorgungsleitung zum Verbraucher zu fördern.

Die Entgasung des Materials erfolgt meist über Dünnschicht-Entgasung, indem das über die Zufluss-Öffnung in den Aufbereitungs-Behälter strömende Material nicht direkt der Füllung des Vorratsbehälters zugeführt wird, sondern auf einen Ableitkörper fließt, dessen - über dem maximalen Füllstand liegende - Oberseite, die Ableitfläche, dazu dient, den Strom des Materials zu verteilen und eine möglichst dünne Schicht aus dem Material auf dieser Ableitfläche entstehen zu lassen, aus der eventuell noch vorhandene Lufteinschlüsse sich umso leichter öffnen, je dünner die Schicht ist, denn je dünner die Schicht ist, umso größer wird die Wahrscheinlichkeit, dass ein Lufteinschluss die Oberseite der Schicht erreicht und damit sich öffnet und das enthaltene Gas, meist Luft, an die Umgebung abgibt.

Zusätzlich herrscht im Luftraum des Aufbereitungs-Behälter oft ein sehr geringer Druck von meist unter 20 mbar, oft unter 5 mbar, oft unter 2 mbar, um das Ausgasen von Luft-Einschlüssen aus der dünnen Schicht zu erleichtern.

Auch andere Entgasungs-Methoden können angewandt werden.

Das Material soll kein Gas, in der Regel Luft, aufweisen, auch nicht in gelöster, nicht sichtbarer, Form und insbesondere keine sichtbaren Gas-Einschlüsse, da selbst noch so kleine Gas-Anteile das aus dem Material hergestellte Produkt beeinträchtigen, beispielsweise die elektrischen Eigenschaften des vergossenen Produktes, etwa die Durchschlagssicherheit einer Vergussmasse um eine elektrische Schaltung, negativ beeinflussen können.

Dabei wird zwischen sichtbaren und nicht sichtbaren Gaseinschlüsse unterschieden, wobei bei den nicht sichtbaren Gaseinschlüssen von "gelösten Gasen" die Rede ist, und selbst in der Fachliteratur strittig ist, ob dies mikroskopisch kleine Blasen sind oder tatsächlich physikalisch-chemisch gelöste Gase, also Gas-Ionen.

Beispielsweise weist Wasser, sei es in einem See im Freien oder in einem Aquarium, einen bestimmten Sauerstoffgehalt auf, der die Bio-Aktivität begrenzt und den die Fische atmen können.

Das **Problem** ist, dass der Gasgehalt bei einem solchem viskosen, vorzugsweise selbst-nivellierenden, Material bisher nicht gemessen wird und damit die Qualität und Quantität der Entgasung nicht nachprüfbar und nicht reproduzierbar ist.

Es gibt allerdings Sensoren, die bestimmte Gas-Arten in einer Flüssigkeit hinsichtlich des Gehalts messen können.

Sogenannte Clark-Sensoren sind amperometrische Sensoren und können den Sauerstoffgehalt in einer Flüssigkeit messen. Sie werden beispielsweise von Firma Mettler-Toledo angeboten und meist in der Pharmazie, der Lebensmitteltechnik oder im Brauwesen angewandt, weil dort häufig der Sauerstoffgehalt von Interesse ist.

Amperometrische Sensoren verbrauchen allerdings während der Messung einen Teil des Gases, insbesondere Sauerstoffs, den sie messen.

Es gibt jedoch auch optisch arbeitende Sensoren für die Messung eines Gas-Anteils in einer Flüssigkeit, bei denen dieser Verbrauch geringer oder Null ist.

Diese optischen Sensoren beruhen meist auf dem Prinzip, dass eine ausgestrahlte definierte elektromagnetische Strahlung von dem Mess-Gas im Material teilweise absorbiert wird, und somit die Differenz zwischen ausgestrahlter Strahlungsmenge und reflektierter Strahlungsmenge ein Maß für den Gehalt an Mess-Gas im Material ist.

Häufig wird dabei nicht auf das Material direkt aufgestrahlt, sondern auf eine Schicht oder einen Layer, der Bestandteil des Sensors ist, dessen reflektierende Eigenschaften durch das Mess-Gas, mit dem es in Kontakt gebracht wird, verändert werden.

Die WO 2019/037970 A1 zeigt eine Ausbringvorrichtung nach dem Oberbegriff von Anspruch 1.

### III. Darstellung der Erfindung

### a) Technische Aufgabe

Es ist daher die Aufgabe gemäß der Erfindung, eine ausbringen-Vorrichtung sowie ein Verfahren zum Bestimmen des Gas-Gehaltes in einem flüssigen oder pastösen, insbesondere des Erreichens oder der Unterschreitung eines vorgegebenen maximal zulässigen Gasgehaltes des Materials, insbesondere während dessen Entgasung, zur Verfügung zu stellen mit dem auch bei noch selbst-nivellierenden, aber hoch-viskosen Materialien die Bestimmung des Gasgehaltes möglich ist selbst bei einem Gas-Anteil von unter 5 Volumen-%, sogar unter 3 Volumen-% oder gar unter 2 Volumen-% im Material.

### b) Lösung der Aufgabe

Diese Aufgabe wird durch die Merkmale der Ansprüche 1 und 8 gelöst. Vorteilhafte Ausführungsformen ergeben sich aus den Unteransprüchen.

Hinsichtlich des Verfahrens wird diese Aufgabe dadurch gelöst, dass das Material vor der Messung, also insbesondere während der Herstellung, Lagerung und Aufbereitung, mit einem Gas-Gemisch in Kontakt gehalten wird oder ohnehin in Kontakt steht, dessen Zusammensetzung bekannt ist und sich auch nur geringfügig ändern kann.

Dies bietet die Möglichkeit, nur den Gehalt einer der Komponenten des Gasgemisches in dem Material, der Mess-Komponente, zu messen, sodass man hierbei frei ist, die am leichtesten messbare Komponente auszuwählen oder eine solche Komponente, die im Gegensatz zu den anderen Komponenten des Gas-Gemisches überhaupt messbar ist.

Von dem Gehalt dieser Mess-Komponente kann dann auf den Gesamtgehalt an Gas, nämlich des gesamten Gas-Gemisches, im Material geschlossen werden.

Der erfinderische Schritt liegt also darin, ein solches Gasgemisch zu wählen, dessen Zusammensetzung man kennt und von der man den Gehalt von mindestens eine Komponente im Material messen kann.

In der Praxis ist das Gas in der Umgebung des Materials ohnehin fast immer Luft, und der Sauerstoffgehalt dieser Luft ist weltweit fast immer der gleiche. sofern er nicht durch Sondereinflussfaktoren wie massivem Verbrauch durch z.B. örtliche Verbrennungsvorgänge verändert wurde.

Vorzugsweise wird also ein Gasgemisch verwendet, welches Sauerstoff enthält, da dessen Gehalt im Material gemessen werden kann. Vorzugsweise wird deshalb eben meist Luft als Gasgemisch gewählt falls dies überhaupt frei wählbar ist.

Als Messverfahren wird vorzugsweise entweder ein amperometrisches Messverfahren, welches die Mess-Komponente während der Messung verbraucht, verwendet oder ein optisches Messverfahren, welche beide auch sehr kleine, nicht mehr sichtbare, Luft-Einschlüsse, also sogenannte gelöste Luft messen.

Bei einem amperometrischen Messverfahren wird üblicherweise ein Elektrolysestrom zwischen zwei Elektroden gemessen, welcher der Konzentration des an der einen Elektrode umgesetzten Stoffes, bei einem Stoffgemisch der Mess-Komponente, direkt proportional ist.

Bei einem sogenannten Clark-Sensor - der ebenfalls zwei Elektroden, meist eine aus Silber und eine aus Platin, in einem einzigen stabförmigen Sensor enthält, aber irreführenderweise häufig im Ganzen als "Clark-Elektrode" bezeichnet wird - wird gelöster Sauerstoff bei einem konstanten elektrischen Potenzial reduziert. Von dem Material sind die Elektroden in der Regel durch eine Membran, die durchlässig für Sauerstoff ist, getrennt.

In den Bereich der amperometrischen Messverfahren fallen auch die Chrono-Amperometrie, und die Bi-Amperometrie.

Bei einem optischen Messverfahren zur quantitativen Bestimmung von Gas in einer Flüssigkeit wird vorzugsweise eine elektromagnetische Strahlung einer solchen Wellenlänge in Richtung des zu bewertenden/messenden Materials gegen das Material gerichtet, die in der Lage ist, mit dieser Frequenz eine zwischen Strahlungsquelle und Material optisch aktive Grenzschicht anzuregen. Durch die Anregung wird Licht eines definierten Emissionsspektrums emittiert und durch einen geeigneten fotosensitiven Sensor detektiert.

Das Vorhandensein von Sauerstoff bzw. der zu messenden Gas-Komponente reduziert das durch die Anregung emittierte Licht in seiner Intensität und/oder seinem Emissionsspektrum.

Folglich korreliert das von der optisch aktiven Grenzschicht des Sensors ausgesandte Licht mit dem Gas-Gehalt des in Kontakt befindlichen Materials.

Dies wird auch als Fluoreszenz-Quenching bezeichnet, wenn hierbei eine optisch aktive, insbesondere fluoreszierende, Schicht als reflektierende Schicht verwendet wird.

Bei einem das Mess-Gas, insbesondere die Mess-Komponente, verbrauchenden Messverfahren wie der Amperometrie wird vorzugsweise die Messung nur dann durchgeführt, wenn davon auszugehen ist, dass zwischen den Elektroden der gleiche Gehalt hiervon vorliegt wie im restlichen Material, insbesondere im Durchschnitt des gesamten restlichen Materials.

Dies kann durch eine ausreichende Strömung an Material im Bereich der Elektroden sichergestellt werden.

Dies gilt ebenso bei optischen Sensoren, bei dem eine Messung nur durchgeführt werden sollte, wenn an der Grenzschicht, die die aufgestrahlte elektromagnetische Strahlung reflektiert, zum Beispiel mittels ausreichender Strömung, der gleiche Gehalt von dem Mess-Gas, insbesondere der Mess-Komponente, wie im restlichen Material vorhanden ist.

Dabei muss bei einem verbrauchenden Messverfahren dafür Sorge getragen werden, dass die Strömung entlang des Sensors so groß ist, dass die Menge des durch die Strömung nach geliefertem Mess-Gases oder der Mess-Komponente größer ist als die in der gleichen Zeiteinheit durch das Messverfahren verbrauchte Menge hiervon.

Häufig herrscht dabei in der Umgebung des Materials mit dem enthaltenen Gas ein reduzierter Druck von unter 100 mbar, insbesondere unter 50 mbar, insbesondere unter 20 mbar, insbesondere unter 10 mbar, was jedoch lediglich bei der Zurverfügungstellung der notwendigen Strömung berücksichtigt werden muss.

Der entsprechende Sensor muss dabei meist in Kontakt mit dem Material stehen, vorzugsweise auch bei einem optischen Sensor, denn die elektromagnetische Strahlung kann nicht direkt auf die Oberfläche des Materials gerichtet und dort reflektiert werden, sondern auf eine optisch aktive Schicht, deren optische Eigenschaften sich durch den Kontakt mit dem Mess-Gas ändert, weshalb diese optische Schicht in Kontakt mit dem Material, in dem sich das Mess-Gas befindet, stehen muss.

Ferner sollte auf der Material-Seite der optisch aktiven Schicht eine sogenannte optische Isolation, etwa schwarzes Silikon, vorhanden sein, die meist im vorderen Ende des Sensors angeordnet ist, um zu verhindern, dass andere elektromagnetische Strahlung, als die vom optischen Sensor aufgestrahlte Strahlung auf das optische Sensorelement auftrifft

Bei einem verbrauchenden Messverfahren wie der Amperometrie ist vorzugsweise das Sensor-Element von dem Material durch eine für das Mess-Gas durchlässigen Membran abgedeckt, wobei die Diffusionsfähigkeit der Membran für das Mess-Gas natürlich möglichst hoch sein sollte, damit das Verhältnis des Gehalts an Mess-Gas auf den beiden Seiten der Membran möglichst nahe bei 1 liegt.

Vorzugsweise sollten die beiden Gehalte in einem bekannten oder etwa durch Kalibrierung ermittelbaren Verhältnis zueinanderstehen, welches bei der Berechnung des Gas-Gehalts berücksichtigt wird.

Üblicherweise wird ja das Material erst dann hinsichtlich des Gasgehaltes als verwendungsfähig eingestuft - was vorzugsweise automatisch erfolgt mittels der Steuerung, die mit dem wenigstens einen Gas-Sensor Signal technisch verbunden ist - wenn ein zulässiger oberer Grenzwert des Gasgehaltes unterschritten ist.

Insbesondere wird das Material auch erst dann dem Verbraucher zugeführt, und/oder wenn stromaufwärts davon an einem Aufbereitungsort eine Aufbereitung in einem Aufbereitungsbehälter durchgeführt wird, erst dann aus dem Aufbereitungsbehälter entnommen, wenn das darin befindliche Material hinsichtlich des Gasgehaltes als verwendungsfähig eingestuft ist.

Der Aufbereitungs-Behälter wird dabei meist chargenweise oder kontinuierlich aus einem Vorratsbehälter mit Material nachgefüllt.

Vorzugsweise wird das Material hinsichtlich des Gasgehaltes erst dann als verwendungsfähig eingestuft, wenn
- ein vorgegebener oberer Grenzwert des Gasgehaltes über eine vorgegebene Kontinuitäts-Zeitspanne unterschritten blieb
   und/oder
- die Abnahme des Gasgehaltes pro Zeiteinheit während einer Abnahme-Zeitspanne geringer war als ein vorgegebener Abnahme-Grenzwert.

Erst dann können die gemessenen Gas-Gehalte als ausreichend stabil betrachtet werden.

Ferner würde bei kontinuierlicher Messung des Gas-Gehaltes zum einen eine enorme Datenmenge anfallen, die zu verarbeiten wäre, zum anderen würde, insbesondere bei aggressivem, insbesondere abrasivem Material und ständiger Anströmung des Sensors durch dieses Material die Kontaktfläche schnell verschlei-ßen.

Deshalb wird gerade bei aggressiven, insbesondere abrasivem, Material vorzugsweise
- der Sensor nur während der Messzeiten dem, insbesondere strömenden, Material ausgesetzt,
- insbesondere außerhalb der Messzeiten das Material am Sensor vorbeigeleitet.

Ferner ist es vorteilhaft, von dem hinsichtlich des Gas-Gehaltes zu überwachenden Material dessen Verhalten vor allem bei der Entgasung möglichst gut zu kennen.

Deshalb wird vorzugsweise bei einem neuen Material zum Kennenlernen des Entgasungs-Verhaltens des neuen Materials neues Material als teach-in-Entgasung vom selben Ausgangs-Zustand des Gas-Gehaltes aus über eine Entgasungs-Zeit entgast wird und der Gasgehalt dabei gemessen, vorzugsweise unter Vorliegen unterschiedlicher Entgasungs-Szenarien in Form von die Entgasung beeinflussenden Entgasungs-Parametern, insbesondere des Umgebungsdrucks und/oder der Material-Temperatur bei der Entgasung und/oder der für das Bewegen des Materials während der Entgasung eingebrachten Energiemenge.

Vorzugsweise wird bei jeder teach-in-Entgasung der minimalste erreichbare Gasgehalt ermittelt, insbesondere daraus der voraussichtliche, bei optimaler Kombination von Entgasungs-Parametern - die bei keinem der teach-in-Entgasungen vorgelegen haben muss - insgesamt minimalste erreichbare Gasgehalt ermittelt oder abgeschätzt. Dies kann anhand des unterschiedlichen Entgasungs-Verhaltens bei den verschiedenen Entgasungs-Szenarien und deren Entgasungs-Parametern erfolgen.

Auf diese Art und Weise können - insbesondere in Kenntnis oder nach Ermittlung des Entgasungs-Verhaltens des Materials -die optimalen Entgasungs-Parameter, etwa der Umgebungsdruck beim Entgasen, und/oder die optimalen Betriebs-Parameter des Verbrauchers, etwa der Durchsatz des Verbrauchers, insbesondere automatisch, ermittelt werden, und dadurch das Material besonders schnell oder besonders wirtschaftlich in einen verwendungsfähigen Zustand aufbereitet werden.

Die Festlegung der optimalen Parameter erfolgt dabei insbesondere in Abhängigkeit von vorgegebenen, vorzugsweise gestaffelt vorgegebenen, Optimierungszielen, etwa dem Energieverbrauch beim Aufbereiten des Materials und/oder dem geforderten Mindest-Durchsatz des Verbrauchers und/oder einer optimal kurzen Aufbereitungs-Zeit für das Material von einem Ausgangs-Zustand in den verwendungsfähigen Zustand.

Gerade hierfür wird der Gas-Gehalt während der Entgasung gemessen, insbesondere jedoch nur in zeitlichen Abständen gemessen, um den wirtschaftlichen Aufwand insbesondere der Daten-Auswertung zu der dadurch erzielten Zunahme an Prozesssicherheit in einem vernünftigen Verhältnis zu halten.

Erfindungsgemäß wird der Gas-Gehalt in der Ausbring-Vorrichtung nicht nur an einer Stelle, sondern an zwei in Strömungsrichtung des Materials beabstandeten Stellen gemessen. Dabei sind zwei Vorgehensweisen möglich, die auch in Kombination miteinander eingesetzt werden können:
Entweder es wird an zwei solchen Stellen gemessen, an denen der Gas-Gehalt gleich sein sollte oder dazwischen eine bestimmte Differenz oder Relation zueinander bestehen sollte - beispielsweise am Auslass eines Aufbereitungs-Behälters einerseits und am Eingang des daraus versorgten Verbrauchers andererseits - sodass die dabei gemessenen Gasgehalte als redundante Messwerte betrachtet werden können und hieraus z.B. auf die korrekte Funktion der Sensoren geschlossen wird oder auf den korrekten Zustand der ausbringen-Vorrichtung zwischen den beiden Messstellen.

Es wird an zwei solchen Stellen gemessen, an denen der Gas-Gehalt unterschiedlich sein sollte - beispielsweise am Material - Einlauf einerseits und am Material-Auslauf eines Aufbereitung-Behälters andererseits - sodass aus dem Unterschied auf z.B. Eigenschaften der Aufbereitungs-Vorrichtung dazwischen, insbesondere auf das Maß der Entgasung dazwischen oder auf Undichtigkeiten dazwischen, geschlossen wird.

Hinsichtlich des Ortes der Messung des Gas-Gehaltes sollte der Gehalt möglichst nahe am Verbrauchsort, insbesondere am Verbraucher, gemessen werden, da der Gas-Gehalt beim Federspeicher Verbrauchen des Materials die wesentliche Information ist.

Wenn das Material entsprechend den Erfordernissen eines Verbrauchers aufbereitet wird und danach abseits vom Aufbereitungs-Ort von einem Verbraucher an einem Verbrauchsort verbraucht, insbesondere ausgebracht, wird, kann
- der Gehalt am Aufbereitungsort gemessen werden
   und/oder
- der Gehalt in einer Zirkulations-Leitung am Aufbereitungsort gemessen werden
   und/oder
- der Gehalt in einer das Material fördernden Fördereinheit wie etwa einer Pumpe gemessen werden.

Eine Ausbring-Vorrichtung für flüssiges oder pastöses Material kann unterschiedliche Module umfassen, üblicherweise
- eine Ausbring-Einheit als Verbraucher
   und/oder
- eine Aufbereitungs-Einheit mit einem Aufbereitungs-Behälter zum Aufbereiten des Materials nach den Erfordernissen eines nachgeschalteten Verbrauchers
   und/oder
- einen Vorrats-Behälter mit Material, insbesondere das vom Hersteller des Materials angelieferte Original-Gebinde,
   und/oder
- eine Förderereinheit zum Fördern des Materials zum Verbraucher, sei es von einem Vorratsbehälter
   und/oder
- Verbindungsleitungen zwischen den Modulen.

Von der Aufbereitungseinheit wird diese Aufgabe dadurch gelöst, dass in dieser Ausbring-Vorrichtung der Gas-Sensor an zwei in Strömungsrichtung des Materials beabsichtigten Positionen angeordnet ist, aufweist zum quantitativen Bestimmen des Gehalts eines Mess-Gases, insbesondere der Mess-Komponente eines Gasgemisches, insbesondere auch in gelöster Form, in dem Material.

Dann wird bevorzugt die Messung möglichst nahe am Verbrauchsort durchgeführt, denn genau dort soll ja ein maximal zulässiger Gasgehalt zuverlässig eingehalten werden.

Ersatzweise kann die Messung in einer Versorgungsleitung vom Aufbereitungsort zum Verbrauchsort gemessen werden, was den Vorteil bietet, dass dort - zumindest periodisch - die bevorzugte Strömung am Gas-Sensor geboten wird.

Das gleiche gilt für die Messung in einer Zirkulationsleitung am Aufbereitungsort, mittels der das vorgehaltene Material am Aufbereitungsort in einem Aufbereitungs-Behälter re-zirkuliert wird.

Bei Messung direkt in einem solchen Aufbereitungsbehälter sollte dies an einer solchen Stelle erfolgen, an der eine ausreichende Strömung herrscht, beispielsweise mittels einer im Aufbereitungsbehälter vorhandenen Bewegungs-Vorrichtung wie etwa eines Rührwerks, oder im Ablauf des Aufbereitungs-Behälters.

Der Gas-Sensor wird dann an der Stelle positioniert, an der - zumindest periodisch - eine ausreichende Strömung herrscht, um den Verbrauch an Mess-Gas nahe am Sensor durch Nachlieferung auszugleichen.

Generell sollte bei einem das Mess-Gas verbrauchenden Gas-Sensor dieser an einer solchen Position in der Ausbring-Vorrichtung angeordnet sein, an der eine Strömung des Materials entlang der Kontaktfläche des Sensors, an der der Gehalt an Mess-Gas gemessen wird oder durch welche Mess-Gas in den Sensor eintritt, vorliegt, zumindest während des Betriebes der Ausbring-Vorrichtung, um die Ausbildung einer stillstehenden oder sich entlang der Kontaktfläche nur wenig bewegenden Grenzschicht an Material zu vermeiden, deren Gas-Gehalt dann nicht repräsentativ für den Gas-Gehalt entfernt von der Grenzschicht wäre.

Dies kann beispielsweise mit einer Schrägstellung der Kontaktfläche zur Strömungsrichtung so, dass die Kontaktfläche durch die Strömung angeströmt wird, erreicht werden, insbesondere wenn der Gas-Sensor und damit dessen Kontaktfläche in einer Rohrleitung oder Schlauchleitung für das Material angeordnet ist

Vorzugsweise weist der dicht verschließbare Aufbereitungs-Behälter in seinem oberen Bereich, also oberhalb des Spiegels an Material, einen Unterdruck-Anschluss auf, sodass im Luftraum oberhalb des Materials und damit im gesamten Aufbereitungs-Behälter der Druck bei unter 900 mbar, besser unter 500 mbar, besser unter 100 mbar, besser bei unter 50 mbar, besser bei unter 20 mbar, besser bei unter 10 mbar, besser bei unter 5 mbar gehalten wird, um das Öffnen von Gas-Einschlüssen im Material zu fördern.

Vorzugsweise wird eine Entnahme des Materials aus dem Aufbereitungsbehälter zur Lieferung an den Verbraucher erst zugelassen, wenn der zulässige maximale Gehalt an Gas unterschritten ist.

Falls es sich bei dem potentiell im Material vorhandenen Gas um Luft handelt, wird vorzugsweise als Gas-Sensor ein den Gehalt an Sauerstoff messender Sensor verwendet, denn daraus kann der Gehalt an Luft insgesamt im Material errechnet werden.

Vorzugsweise wird der Gas-Sensor in einer das Material kontaktierenden Stellung eingesetzt, was bei einigen Sensor-Arten ohnehin unumgänglich ist.

Bevorzugt wird ein amperometrischer Gas-Sensor verwendet, insbesondere ein Clark-Sensor, da derartige Sensoren als Zukaufteil verfügbar sind.

Ein solcher verbrauchender Gas-Sensor ist vorzugsweise durch eine Membran, die für das Mess-Gas durchlässig ist, vom Material getrennt.

Die Diffusionsfähigkeit der Membran für das Mess-Gas muss dabei so groß sein, dass sich ein im wesentlichen konstanten Gradient des Gehaltes an dem Mess-Gas zwischen den beiden Seiten der Membran einstellt, der möglichst nahe bei 1 liegen sollte.

Alternativ steht der Gehalt beidseits der Membran in einem bekannten Verhältnis zueinander und kann bei der Bestimmung des Gesamt-Gehaltes an Gas im Material berücksichtigt werden.

Alternativ kann ein optischer Sensor verwendet werden, dessen abgegebene Strahlung durch den Gas-Anteil, insbesondere dessen Mess-Komponente im Material reflektiert wird.

Vorzugsweise wird dabei zwischen dem optischen Sensor und dem Material eine optische Isolation vorgesehen, damit nur von dem optischen Sensor ausgestrahlte elektromagnetische Strahlung in reflektierter Form an dessen Sensor-Element ankommt, und kein Fremd-Licht.

### c) Ausführungsbeispiele

Ausführungsformen gemäß der Erfindung sind im Folgenden beispielhaft näher beschrieben. Es zeigen:
- **Figur 1:**: eine beispielhafte Ausbring-Vorrichtung mit möglichen Positionen für die Anbringung eines Gas-Sensors,
- **Figur 2:**: einen Vertikalschnitt durch einen Aufbereitungs-Behälter,
- **Figur 3a:**: eine Funktionsskizze für einen optischen Sensor,
- **Figur 3b:**: eine Funktions-Skizze für einen amperometrischen Sensor.

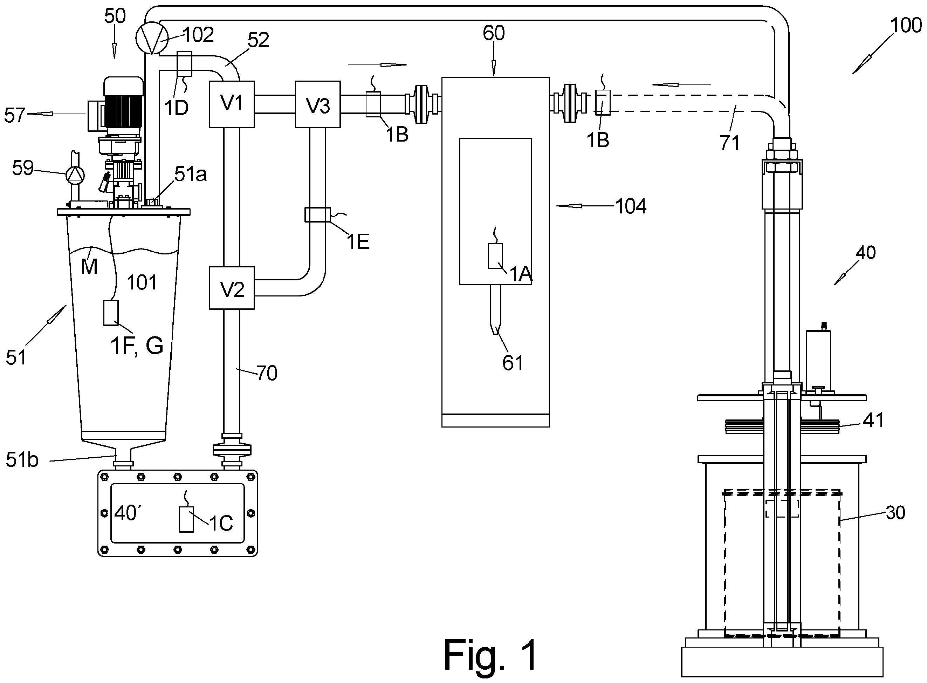

**Figur 1** zeigt eine Ausbring-Vorrichtung 100 mit einer Ausbring-Einheit 60 als Verbraucher der Ausbring-Vorrichtung 100, indem dort über eine Ausbring-Düse 61 Material dosiert auf ein - nicht dargestelltes - Bauteil abgegeben wird.

Die linke und rechte Anschlussseite der Ausbring-Einheit 60 sind - in dieser Form hypothetisch - eher Alternativen als gleichzeitig vorhanden:
Die rechte Anschlussseite zeigt, wie Material M mittels einer Förder-Einheit 40 direkt, also ohne Aufbereitung dazwischen, aus einem Vorratsbehälter 30 für das Material M entnommen und der Ausbring-Einheit 60 über eine Verbindungsleitung 71 0zugeführt wird, in diesem Fall mittels einer Fassfolge-Entleer-Vorrichtung 40 mit einer Fassfolge-Platte 41, wobei der Vorratsbehälter 30 das Original-Gebinde, ein sogenannter Hobbock, ist, in dem das Material vom Hersteller angeliefert wird.

Die linke Anschlussseite zeigt den weitaus häufigeren Fall, dass der Verbraucher 60 aus einer Aufbereitungs-Einheit 50 - welche aus dem Vorrats-Behälter 30 nachgefüllt wird - mit aufbereiteten Material M versorgt wird:
Dort wird das Material M in einem Aufbereitungs-Behälter 51 aufbereitet, beispielsweise durchmischt und entgast, und von dort über eine Förder-Einheit 40' in Form meist einer Pumpe und eine Verbindungsleitung 70 dem Verbraucher 60 zugeführt.

Zum Entgasen verfügt der Aufbereitungsbehälter 51 über einen Unterdruck-Anschluss, der mit einer Unterdruck-Quelle 59 in Verbindung steht. Das Material M wird über eine Zufluss-Öffnung 51a eingebracht und über eine Abfluss-Öffnung 51b daraus entnommen. Der Zufluss zur Zufluss-Öffnung 51a erfolgt dabei meist aus dem Vorratsbehälter 30 wie in der rechten Bildhälfte dargestellt.

Zusätzlich kann eine Zirkulationsleitung 52 vorhanden sein, über die ständig mittels der Pumpe 40' - hier der gleichen Pumpe 40 Strich, die auch den Transport des Materials über die Verbindungsleitung 70 zum Verbraucher 60 bewirkt - aus der Abfluss-Öffnung 51b entnommenes Material wieder über die Zufluss-Öffnung 51a im oberen Bereich in den Aufbereitungsbehälter 51 eingegeben wird. Die Zirkulationsleitung 52 ist teilweise identisch mit der Verbindungsleitung 70 und mittels eines Ventils V1 kann - vorzugsweise automatisch - die Zuführung des Materials M wahlweise zum Verbraucher 60 oder zur Zufluss-Öffnung 51a gesteuert werden.

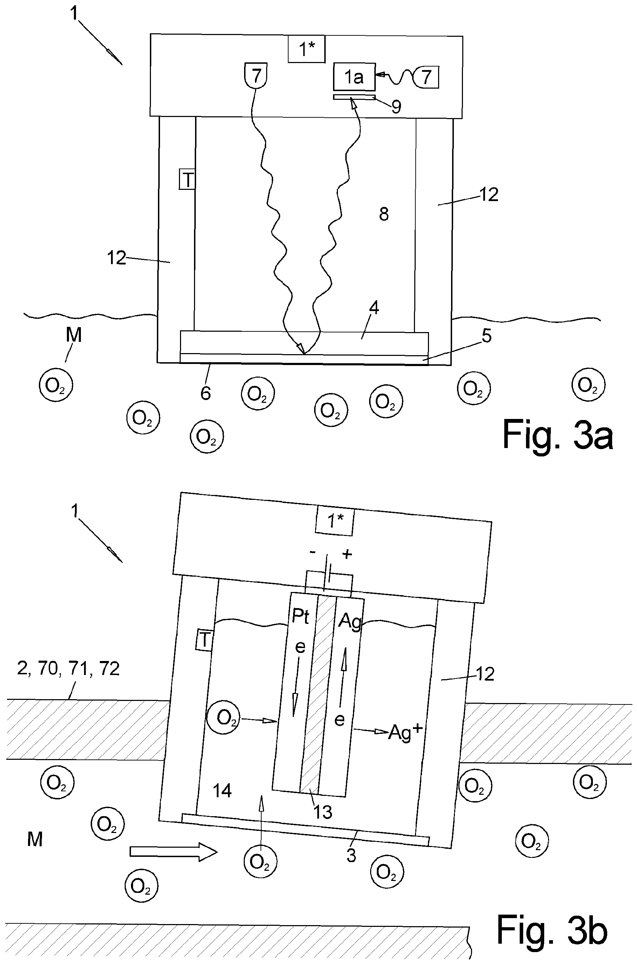

**Figur 1** zeigt, an welchen Stellen innerhalb einer solchen Ausbring-Vorrichtung 100 ein Gas-Sensor 1 - der in der Regel in einer Sensor-Aufnahme 2 aufgenommen ist, die beispielsweise über eine Durchfluss-Öffnung verfügen kann und in eine Rohrleitung eingesetzt werden kann, wie in **Figur 3** wie ersichtlich - angeordnet werden könnte:
Prinzipiell besteht das Bestreben darin, möglichst nah an der Ausbringungsstelle, also der Ausbring-Düse 61 den Gas-Gehalt zu messen, denn der Gas-Gehalt dort ist entscheidend.

Vorzugsweise wird deshalb der Gas-Sensor 1 direkt in der Ausbring-Einheit 60, also in der Position A, angeordnet werden, vorzugsweise in dessen interner Verrohrung bzw. Durchflusskanälen, möglichst kurz vor der Ausbring-Düse 61 und damit dem - bei einem aus mehreren Komponenten bestehenden Material M meist nicht dargestellten, daran befestigten - statischen oder dynamischen Mischer-Rohr.

Sofern dies nicht möglich oder nicht erwünscht ist, könnte ein solcher Gas-Sensor 1 in der Verbindungsleitung 70 oder 71, also in Position B, angeordnet werden, dann vorzugsweise möglichst nah am Anschluss der Ausbring-Einheit 60 hin.

Der Gas-Sensor 1 kann jedoch auch in der Zirkulationsleitung 52, vorzugsweise stromabwärts des Ventils V1, als Position D angeordnet sein.

Da sich die Pumpe 40' in aller Regel stromaufwärts des Ventils V1 und meist unmittelbar anschließend, also ohne vorherige Verzweigung, an die Auslauf-Öffnung 51 b des Aufbereitung-Behälters 51 befindet, kann der Gas-Sensor 1 als Position C auch in der Pumpe 40`, beispielsweise in deren Einlauf-Stutzen oder Auslauf-Stutzen, angeordnet sein
Gerade wenn aggressive, zum Beispiel abrasive Materialien M verarbeitet werden sollen, kann es auch sinnvoll sein, zu der Verbindungsleitung 70 von der Aufbereitung-Einheit 50 zum Verbraucher 60 eine Bypass-Leitung 72 vorzusehen, wobei zumindest an der Abzweigung von der Verbindungsleitung 70 ein schaltbares Ventil V2 vorgesehen ist, vorzugsweise auch an deren Mündung zurück in die Verbindungsleitung 70.

Dann kann in dem Bypass 72 ein Gas-Sensor 1 als Position E vorgesehen werden, und der Strom aus Material wird durch entsprechendes schalten des Ventils V2 und gegebenenfalls auch V3 nur zu den Zeiten der Messung über den dortigen Gas-Sensor 1 geleitet, der außerhalb der Messzeiten somit nicht durch das abrasive Material M verschlissen wird.

Sofern der Gas-Sensor 1 woanders untergebracht werden soll, kommt hierbei vorzugsweise die Aufbereitungs-Einheit 50 in Frage, und vorzugsweise der Aufbereitungsbehälters 51.

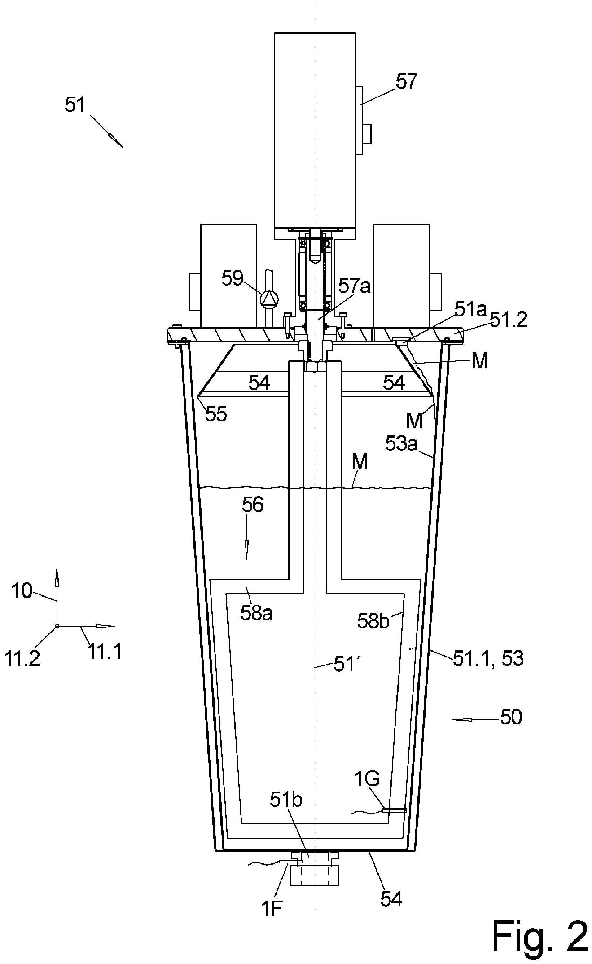

Ein solcher Aufbereitungsbehälter 51 ist in **Figur 2** im Schnitt dargestellt, bestehend aus einem oben offenen Topf 51.1 und diesen an der Oberseite dicht verschließenden Deckel 51.2.

Im Inneren dieses Aufbereitungsbehälters 51 rotiert ein Rührer 56 mit in diesem Fall zwei auf einander gegenüberliegenden Seiten bzgl. der Rotationsachse angeordneten Rührflügeln 58a, b, welche in diesem Fall drehfest an einer Motorwelle 57a mit ihrem oberen Ende befestigt sind und sich von dort nahe der vertikalen Symmetrieachse 51' des in der Regel in der Aufsicht betrachtet notationssymmetrischen Behälters 51 nach unten erstrecken, aber im unteren Bereich weiter nach außen erstrecken und mit der Außenkante nahe an der Innenfläche der Wandung 53 des Topfes 51.1 entlanglaufen, wenn sie rotierend angetrieben werden.

Die Motorwelle 57a ist die Antriebswelle eines Motors 57, der den Rührer-Antrieb darstellt, und auf der Oberseite des Deckels 51.2 angeordnet ist, sodass die Motorwelle 57a diesen von oben nach unten durchläuft.

Im Boden ist eine Abfluss-Öffnung 51b zentral auf der Symmetrieachse 51' dargestellt.

In der Abfluss-Öffnung 51 b und/oder deren Anschlussstutzen ist direkt im Auslauf unter dem Boden ein Gas-Sensor 1 an Position F dargestellt, der dann direkt am Auslauf den Gasgehalt im Material messen würde was auf den Vorteil hat, dass wie bei Anordnung in einer Leitung eine Strömung an Material M an der Kontaktfläche des Gas-Sensors 1 entlang strömt.

Aus dem gleichen Grund kann ein Gas-Sensor 1 als Position G an einem der Rührflügel 58b befestigt sein, dann vorzugsweise mit seiner Kontaktfläche radial nach außen oder nach innen über den Rührflügel 58 b vorstehend und möglichst tief über dem Boden, um auch bei niedrigen Füllstand im Aufbereitungs-Behälter 51 noch den Gasgehalt im Material M messen zu können.

Nachteilig ist dann, dass eine Stromversorgung sowie eine signaltechnische Verbindung von dem rotierenden Rührflügel zur Steuerung gewährleistet werden muss.

Außer dem Unterdruck-Anschluss zur Unterdruck-Quelle 59 ist im Deckel 51.2 in diesem Fall eine, manchmal aber auch zwei Zufluss-Öffnungen 51a vorgesehen, beispielsweise um zwei getrennte Komponenten in den Aufbereitungsbehälter 51 einbringen und darin mischen zu können, wobei der Zufluss über je ein darüber sitzendes Einlassventil geregelt wird.

Das Material M läuft aus der Zufluss-Öffnung 51a zunächst auf die Oberseite eines Kegelstumpf-förmigen, vorzugsweise ebenfalls rotierenden, etwa mit den dem Rührer 56 mit rotierenden, Ableitkörper 54 und bildet dort eine dünne Schicht aus, die über dessen untere Umfangskante als Abtropfkante 55 herabfließt.

Da der Topf 51.1 sich nach oben konisch erweitert und sich die Abtropfkante 55 nahe der Innenfläche 53a der Umfangswand 53 des Topfes 51.1 befindet, läuft anschließend die dünne Schicht von der Abtropfkante 55 an der Innenfläche der Umfangswand weiter abwärts, und das Ausbilden dieser dünnen Schichten begünstigt das Entgasen des Materials zusätzlich zu dem in dem Aufbereitungs-Behälter 51 herrschenden Unterdruck.

Die **Figuren 3a**,**b** zeigen die Funktion-Prinzipien zweier infrage kommender Gas-Sensoren 1:
**Figur 3a** ist eine Prinzip-Darstellung eines optischen Gas-Sensors 1 zur Sauerstoffmessung:
Das Kernelement ist eine optisch aktive Schicht 5, die bei Kontakt mit dem Mess-gas, hier Sauerstoff, ihre optischen Eigenschaften verändert.

Beispielsweise kann die optisch aktive Schicht 5 eine reflektierende oder gar fluoreszierende Schicht sein, deren reflektierende Eigenschaften oder Fluoreszenz-Eigenschaften sich umso stärker ändern, mit je mehr Sauerstoff pro Zeiteinheit diese optisch aktive Schicht 5 in Kontakt gerät.

Zu diesem Zweck wird die optische Schicht 5 von der einen Seite her mit einer anregenden elektromagnetischen Strahlung, hier sichtbarem Licht abgegeben von einer LED 7, beaufschlagt - vorzugsweise geführt durch eine Glasfaser 8 oder einen Strang aus Glasfasern 8 - und das daraufhin von der optischen Schicht 5 reflektierte und in die gleiche Richtung zurückgeworfene, z.B. fluoreszierte Licht von einem Sensorelement 1a detektiert. Dabei kann das am Sensorelement 1a eintreffende reflektierte Licht mittels eines optischen Filters 9 gefiltert werden auf die von der anregenden LED abgegebene Frequenz oder Frequenzbereich.

Vorzugsweise wird das Sensorelement 1a auch von einer Referenz-LED 7 bestrahlt, die der anregenden LED 7 entspricht und Licht in der gleichen Menge und mit dem gleichen Frequenz bzw. Frequenzbereich abgibt, sodass die Sensorelement 1a bei abwechselndem Messen des von der Referenz-LED 7 empfangenen Lichts und des von der optisch aktiven Schicht 5 empfangenen Lichts - sei es hinsichtlich Wellenlänge oder Menge - die Messergebnisse an die angeschlossene Steuerung 1* weitergibt, die die Differenz umrechnet in einen Wert, der den Gehalt an Sauerstoff in dem Material repräsentiert, welches bei der Messung mit der optisch aktiven Schicht 5 in Kontakt ist.

Damit von der der Anregungsseite gegenüberliegenden Seite kein Fremdlicht die optisch aktive Schicht 5 erreichen kann, ist diese auf der von der optisch aktiven Schicht abgewandten Seite, der Material-Seite, mit einer optisch isolierenden Schicht 6 beschichtet (oder hat selbst optisch isolierende Eigenschaften), die jedoch für die Sauerstoffmoleküle durchlässig ist.

Konstruktiv wird die optisch aktive Schicht 5 abgestützt von einer für das anregenden reflektierte Licht vollständig durchlässigen Stützplatte 4 meist aus Glas, wobei sich die optisch isolierende Schicht 6 auf der von der Stützplatte 4 abgewandten Material-befindet.

Diese Schichtanordnung ist an ihrem Umfang dicht am oder im vorderen Endbereich eines Führungsrohres 12 angeordnet, welches auch die optische Glasfaser 8 oder den Glasfaser-Strang 8 führt und umfänglich schützt.

Am anderen Ende des Führungsrohres 12 oder noch im Führungstor 12 im anderen Endbereich sind die elektrisch betriebenen Komponenten untergebracht, also die beiden LEDs 7, das Sensorelement 1a mit vorgelagertem optischen Filter 9 sowie die Steuerung 1*.

Zusätzlich kann ein Temperatursensor T, positioniert vorzugsweise am oder im Innenumfang des Stützrohres 12, vorhanden sein, um die Betriebstemperatur des optischen Sensors zu überwachen, da das Messergebnis Temperaturabhängig ist und eventuell auch hinsichtlich der Temperatur während des Messvorganges kalibriert werden muss.

**Figur 3b** ist eine Prinzip-Darstellung eines Clark-Sensors, also eines amperometrischen Gas-Sensors 1, zur Sauerstoffmessung:
Dabei ragen eine - meist aus Silber bestehende - Anode und eine - meist aus Platin bestehende - Kathode, die mittels eines Isolators 13 elektrisch gegeneinander isoliert sind, in einen in der Regel flüssigen Elektrolyt 14, in dem sich Sauerstoff-Moleküle befinden. Anode oder Kathode sind über eine Spannungsquelle miteinander verbunden, deren Betriebsspannung sich umso stärker ändert, je stärker die im Sensor 1 ablaufende chemische Reaktion ist, nämlich
An der Kathode: O₂ + 4e⁻ +2 H₂O = 4 OH-
An der Anode: 4 Ag +4 Cl = 4 Ag Cl + 4e⁻

Die angeschlossene Steuerung 1* misst die auftretende Veränderung der Betriebsspannung und wandelt sie in einen für den Sauerstoffgehalt im Material M repräsentativen Wert um.

Konstruktiv befinden sich Anode und Kathode sowie das Elektrolyt 14 in einem geschlossenen Stützrohr 12, in dessen vorderen Endbereich eine dessen Stirnseite dicht verschließende, für den Sauerstoff im Material jedoch durchlässige, Membran 3 eingebracht ist und dessen hinteres Ende verschlossen ist und nur von den hinteren Enden der Elektroden oder deren elektrischen Zuleitungen durchlaufen wird.

In Figur 3b ist der Sensor 1 mit seiner axialen Richtung, also der Verlaufsrichtung seines Stützrohres 12, quer, aber nicht genau lotrecht in die Wanderung einer Sensor-Aufnahme 2 oder einer Leitung 70,71, 72 dicht eingebaut sondern in einer solchen Schräglage, dass die durch die Außenseite der Membran 3 gebildete Kontaktfläche, insbesondere mit einer Neigung von einigen Grad, der an der Stelle des Sensors 1 herrschenden Strömung an Material M in der Leitung oder der Sensor-Aufnahme 2 entgegensteht.

Auch hier kann ein Temperatur-Sensor T vorhanden sein, vorzugsweise in dem Innenumfang des Stützrohres 2, der die Betriebstemperatur des Gas-Sensors 1 überwacht und an die Steuerung 1* meldet, sodass man daraufhin den Gas-Sensor 1 neu kalibrieren oder den repräsentativen Wert an die gemessene Temperatur anpassen kann.

### BEZUGSZEICHENLISTE

- 1, 1.1, 1.2: Gas-Sensor
- 1*: Steuerung
- 1a, b: Sensor-Element
- 2: Sensor-Aufnahme
- 3: Membran
- 4: Stützplatte, Glasplatte
- 5: optisch aktive Schicht
- 6: optisch isolierende Schicht
- 7: LED
- 8: Glasfaser, Glasfaserbündel
- 9: optischer Filter
- 10: Vertikale
- 11.1, 11.2: horizontale Querrichtung
- 12: Stützrohr

- 30: Vorratsbehälter

- 40: Fördereinheit, Fassfolge-Entleer-Vorrichtung
- 40': Fördereinheit, Pumpe
- 41: Fassfolge-Platte
- 50: Aufbereitungs-Einheit
- 51: Aufbereitungsbehälter
- 51.1: Topf
- 51.2: Deckel
- 51': Symmetrieachse, Zentrum, Rotationsachse
- 51a: Zufluss-Öffnung
- 51b: Abfluss-Öffnung
- 52: Zirkulationsleitung
- 53: Wandung
- 53a: Innenfläche
- 54: Ableitkörper
- 55: Kante, Abtropfkante
- 56: Rührer
- 57: Rührer-Antrieb, Motor
- 57a: Motorwelle, Antriebswelle
- 58a, b, c: Rührflügel
- 59: Unterdruck-Quelle
- 60: Verbraucher, Ausbring-Einheit
- 61: Ausbring-Düse

- 70: Verbindungsleitung
- 71: Verbindungsleitung
- 72: Bypass-Leitung

- 100: Ausbring-Vorrichtung
- 100*: Steuerung

- A - G: Position

## Patentansprüche

1. **Ausbring-Vorrichtung** (100) zum Ausbringen eines flüssigen oder pastösen Materials (M), umfassend an Modulen
- einen Verbraucher (60),
- eine Aufbereitungs-Einheit (50) mit einem Aufbereitungs-Behälter (51) zum Aufbereiten des Materials (M) und
- eine Fördereinheit (40, 40') zum Fördern des Materials (M)
wobei
die Ausbring-Vorrichtung (100) mindestens einen Gas-Sensor (1) zum Ermitteln des Gehaltes des Mess-Gases (G) im Material (M) aufweist,
**dadurch gekennzeichnet, dass**
der Gas-Sensor (1) an wenigstens zwei in Strömungsrichtung des Materials (M) beabstandeten Positionen angeordnet ist, welche ausgewählt sind von:
- in der Aufbereitungs-Einheit (50), ortsfest als Position (F) oder an einem Rührflügel, (58b) als Position (G);
- in einer Zirkulations-Leitung (52) an der Aufbereitungs-Einheit (50) als Position (D),
- in oder an dem Verbraucher (60) als Position (A),
- an oder in der Fördereinheit (40, 40') als Position (C),
- in Verbindungsleitungen (70) dazwischen als Position (B).

2. Ausbring-Vorrichtung nach Anspruch 1,
**dadurch gekennzeichnet, dass**
der Gas-Sensor (1) ein Sauerstoff in einem Gasgemisch messender optischer Fluoreszenz-Quenching-Sensor ist
und/oder
- die Anbring-Vorrichtung (100) zum Ausbringen eines flüssigen oder pastösen, selbst-nivellierenden, Materials (M) dient und der Verbraucher (60) eine Ausbring-Einheit (60) zum Ausbringen des Materials (60) ist,
und/oder
- die Aufbereitungs-Einheit (50) mit einem Aufbereitungs-Behälter (51) zum Aufbereiten des Materials (M) nach den Erfordernissen des Verbrauchers (60) ausgebildet ist, in welchem insbesondere das Material in Kontakt mit einem bekannten, definierten Gas oder Gasgemisch ist,
und/oder
- die Fördereinheit (40, 40') zum Fördern des Materials (M) von einem Vorratsbehälter (30) zum Verbraucher (60) ausgebildet ist,
wobei der
Gas-Sensor (1) zum Ermitteln des Gehaltes des Mess-Gases (G) eine zu messende Mess-Komponente eines Gas-Gemisches ist, im Material (M) ausgebildet ist.

3. Ausbring-Vorrichtung nach einem der vorhergehenden Ansprüche,
**dadurch gekennzeichnet, dass**
- der Gas-Sensor (1) ein Sauerstoff messender Gas-Sensor (1) ist.
und/oder
- der Gas-Sensor (1) ein das Material kontaktierender Sensor (1) ist
und/oder
- der Gas-Sensor (1) ein das Mess-Gas, insbesondere die zu messende Mess-Komponente eines Gas-Gemisches, verbrauchender Sensor (1) ist
und/oder
- der Gas-Sensor (1) ein den Sauerstoffgehalt in dem Material (M) messender optischer Sensor (1.1) ist.

4. Ausbring-Vorrichtung nach Anspruch 3,
**dadurch gekennzeichnet, dass**
der optische Sensor (1.1) eine optisch aktive Schicht zwischen der Strahlungsquelle und dem Material aufweist, die durch die auftreffende Strahlung angeregt wird und eine (Sekundär-)Strahlung, insbesondere eines definierten Emissionsspektrums, abgibt, welche von einem geeigneten Sensor detektiert wird, wobei das Vorhandensein des Mess-Gases im Material das durch die Anregung emittierte Licht in seiner Intensität und/oder seinem Emissionsspektrum verändert, insbesondere reduziert.

5. Ausbring-Vorrichtung nach einem der vorhergehenden Ansprüche,
**dadurch gekennzeichnet, dass**
- der Gas-Sensor (1) ein amperometrischer Sensor (1.2) ist,
- insbesondere der Gas-Sensor (1.2) ein den Sauerstoffgehalt in einer Flüssigkeit messender Clark-Sensor (1.2) ist,
- insbesondere die Sensor-Elemente (1a, b) am amperometrischen Sensor (1.2), insbesondere am Clark-Sensor, durch eine Membran (3), die für das Mess-Gas durchlässig ist, abgedeckt sind.

6. Ausbring-Vorrichtung nach einem der vorhergehenden Ansprüche,
**dadurch gekennzeichnet, dass**
insbesondere bei einem das Mess-Gas verbrauchender Sensor (1)
- dieser an einer solchen Position in der Ausbring-Vorrichtung angeordnet ist, an der eine Strömung des Materials (M) entlang der Kontaktfläche des Sensors (1) zum Material, an der der Gehalt an Mess-Gas gemessen wird oder durch welche Mess-Gas in den Sensor eintritt, vorliegt, zumindest während des Betriebes der Ausbring-Vorrichtung,
- insbesondere mit einer Schrägstellung der Kontaktfläche zur Strömungsrichtung so, dass die Kontaktfläche durch die Strömung angeströmt wird,
- insbesondere in einer Rohrleitung oder Schlauchleitung für das Material (M).

7. Ausbring-Vorrichtung nach einem der vorhergehenden Ansprüche,
**dadurch gekennzeichnet, dass**
- im Aufbereitungs-Behälter eine Bewegungs-Vorrichtung (56), insbesondere ein Rührwerk (56), vorhanden ist,
- die Bewegungs-Vorrichtung (56) in der Lage ist, eine so starke Strömung des Materials (M),
- insbesondere an der Grenzschicht zum Gas-Sensor hin, aufrechtzuerhalten, dass die durch die Strömung stattfindende Nachlieferung des Mess-Gases, insbesondere der Mess-Komponente, an die Kontaktfläche des Sensors (1.2) größer ist als der Verbrauch des Mess-Gases durch den amperometrischen Gas-Sensor (1.2),
- insbesondere der obere Bereich des Innenraumes des Aufbereitungs-Behälters (51) und/oder des Vorrats-Behälters (30) mit einer Unterdruck-Quelle (59) in Verbindung bringbar ist,
- die Unterdruckquelle (59) in der Lage ist, das Gas-Gemisch im Gasraum des Aufbereitungs-Behälters oberhalb des Materials auf einem Druck von unter 900 mbar, besser unter 500 mbar, besser unter 100 mbar, besser unter 50 mbar, besser unter 20 mbar, besser unter 10 mbar, besser unter 5 mbar, zu halten.

8. **Verfahren** zum Bestimmen des Gas-Gehaltes in einem flüssigen oder pastösen Material (M),
wobei
- der Gehalt an einem solchen Mess-Gas in dem Material (M) gemessen wird,
- von dem bekannt ist, dass das Material (M) vorher damit in Kontakt war und man im Falle eines Gasgemisches dessen Zusammensetzung kennt,
- im Fall eines Gas-Gemisches aus dem Gehalt dieser Mess-Komponente auf den Gehalt des Gasgemisches insgesamt im Material (M) geschlossen wird,
wobei
- das Material entsprechend den Erfordernissen eines Verbrauchers (60) aufbereitet wird und danach abseits vom Aufbereitungsort von einem Verbraucher (60) an einem Verbrauchsort verbraucht wird,
**dadurch gekennzeichnet, dass**
- der Gehalt des Mess-Gases an wenigstens zwei in Strömungsrichtung des Materials (M) beabstandeten Positionen gemessen wird, welche ausgewählt sind von:
- einer Position (A), wo der Gehalt möglichst nahe am Verbraucher (60) gemessen wird,
- einer Position (F, G), wo der Gehalt am Aufbereitungsort gemessen wird,
- einer Position (D), wo der Gehalt in einer Zirkulations-Leitung (70) am Aufbereitungsort gemessen wird,
- einer Position (C), wo der Gehalt in einer das Material (M) fördernden Fördereinheit (40) gemessen wird,
- einer Position (B) in Verbindungsleitungen (70) dazwischen.

9. Verfahren nach Anspruch 8,
**dadurch gekennzeichnet, dass**
das Verfahren im Rahmen einer Ausbring-Vorrichtung (100) nach einem der vorhergehenden Vorrichtungs-Ansprüche ausgeführt wird,
und/oder
das Gasgemisch Sauerstoff enthält und ein optisches Fluoreszenz-Quenching - Messverfahren angewendet wird,
und/oder
als Gasgemisch ein solches verwendet wird, welches Sauerstoff enthält, insbesondere Luft,
- ein optisches Messverfahren verwendet wird,
- insbesondere Fluoreszenz-Quenching
und/oder
- ein amperometrisches Messverfahren verwendet wird,
- damit insbesondere auch der Gehalt an gelöstem Sauerstoff gemessen wird.

10. Verfahren nach einem der Ansprüche 8 bis 9,
wobei ein Sensor-Prinzip angewandt wird, bei welchem das Mess-Gas, dessen Gehalt gemessen wird, zumindest teilweise durch den Sensor (1), insbesondere einen amperometrischen Sensor (1.2), verbraucht wird,
**dadurch gekennzeichnet, dass**
- eine Messung dann durchgeführt wird, wenn davon auszugehen ist, dass in der Grenzschicht des Materials (M) am Sensor (1) und/oder dem Sensor-Element des Sensors (1) der gleiche Gehalt an Mess-Gas vorliegt wie durchschnittlich im restlichen Material (M) innerhalb des Behälters oder des Moduls, in dem gemessen wird,
- oder ein Gehalt, der für den Gehalt an Mess-Gas im restlichen Material (M) innerhalb des Behälters oder des Moduls, in dem gemessen wird, repräsentativ ist,
- insbesondere für den durchschnittlich im gesamten Material vorhandenen Gehalt repräsentativ ist.

11. Verfahren nach einem der Ansprüche 8 bis 10,
**dadurch gekennzeichnet, dass**
- das Gas-Gemisch mit einem Unterdruck von unter 900 mbar, besser von unter 500 mbar, besser von unter 100 mbar, besser von unter 50 mbar, besser von unter 20 mbar, besser von unter 10 mbar, besser von unter 5 mbar gehalten wird und insbesondere der Unterdruck in Kontakt mit dem Material (M) gehalten wird
und/oder
- das Messen des Gehalts an Mess-Gas mittels eines das Material (M) kontaktierenden Gas-Sensors (1) durchgeführt wird und
- der Gas-Sensor (1) so angeordnet und eingesetzt wird, dass die Ausbildung einer relativ zum Sensor (1) sich sehr langsam bewegenden oder gar stillstehenden Grenzschicht aus Material (M) verhindert wird.

12. Verfahren nach einem der Ansprüche 8 bis 11, **dadurch gekennzeichnet, dass**
eine so starke Strömung des Materials (M), insbesondere an der Grenzschicht zum Gas-Sensor (1) hin, während der Messung aufrechterhalten wird, dass die durch die Strömung stattfindende Nachlieferung des Mess-Gases an die Kontaktfläche des Sensors (1) größer ist als der Verbrauch des Mess-Gases durch den verbrauchenden Gas-Sensor (1).

13. Verfahren nach einem der Ansprüche 8 bis 12, **dadurch gekennzeichnet, dass**
- die Sensor-Elemente des Gas-Sensors (1) von einer Membran (3) abgedeckt sind, die für die Mess-Komponente des Gases durchlässig ist,
- insbesondere die Diffusionsfähigkeit der Membran (3) für das Mess-Gas bekannt ist, sodass auch das Verhältnis des Gehalts an Mess-Gas auf den beiden Seiten der Membran (3) bekannt ist oder bestimmt werden kann, insbesondere mit Hilfe eines Kalibrier-Vorganges.

14. Verfahren nach einem der Ansprüche 8 bis 13,
wobei das Verfahren zum Bestimmen des Gas-Gehaltes in einem flüssigen oder pastösen, selbst-nivellierenden Material (M) dient,
- insbesondere des Erreichens oder der Unterschreitung eines vorgegebenen maximal zulässigen Gasgehaltes des Materials (M), insbesondere während dessen Entgasung,
wobei
- der Gehalt an einer Mess-Komponente des Gasgemisches in dem Material (M) gemessen wird,
- von dem bekannt ist, dass das Material (M) vorher damit in Kontakt war und man im Falle des Gasgemisches dessen Zusammensetzung kennt,
- im Fall eines Gas-Gemisches aus dem Gehalt dieser Mess-Komponente auf den Gehalt des Gasgemisches insgesamt im Material (M) hochgerechnet wird.

15. Verfahren nach einem der Ansprüche 8 bis 14, **dadurch gekennzeichnet, dass**
- das Materials (M) erst, insbesondere automatisch, hinsichtlich des Gasgehaltes als verwendungsfähig eingestuft wird, insbesondere Material dem Verbraucher (60) erst zugeführt wird, wenn ein zulässiger oberer Grenzwert des Gasgehaltes unterschritten ist,
- insbesondere, wenn am Aufbereitungsort die Aufbereitung in einem Aufbereitungsbehälter (51) durchgeführt wird,
- der Aufbereitungs-Behälter (51) chargenweise oder kontinuierlich aus einem Vorratsbehälter (30) mit Material (M) nachgefüllt wird, eine Entnahme aus dem Aufbereitungs-Behälter (51) für den Verbraucher (60) erst, insbesondere automatisch, zugelassen wird, wenn das Material (M) hinsichtlich des Gasgehaltes als verwendungsfähig eingestuft ist.

16. Verfahren nach einem der Ansprüche 8 bis 15, **dadurch gekennzeichnet, dass**
Material (M) hinsichtlich des Gasgehaltes erst dann als verwendungsfähig eingestuft wird, wenn
- ein vorgegebener oberer Grenzwert des Gasgehaltes über eine vorgegebene Kontinuitäts-Zeitspanne unterschritten blieb
und/oder
- die Abnahme des Gasgehaltes während einer Abnahme-Zeitspanne geringer war als ein vorgegebener Abnahme-Grenzwert
insbesondere
bei abrasivem Material
- der Sensor (1) nur während der Messzeiten dem, insbesondere strömenden, Material (M) ausgesetzt wird,
- insbesondere außerhalb der Messzeiten das Material am Sensor ((1) vorbeigeleitet wird.

17. Verfahren nach einem der Ansprüche 8 bis 16, **dadurch gekennzeichnet, dass**
bei einem neuen Material zum Kennenlernen des Entgasungs-Verhaltens des neuen Materials
- neues Material vom selben Ausgangs-Zustand aus über eine EntgasungsZeit entgast wird und der Gasgehalt dabei gemessen wird unter Vorliegen unterschiedlicher Entgasungs-Szenarien in Form von die Entgasung beeinflussenden Parametern, insbesondere des Umgebungsdrucks und/oder der Material-Temperatur bei der Entgasung und/oder der für das Bewegen des Materials während der Entgasung eingebrachten Energiemenge,
- insbesondere dabei der minimalste erreichbare Gasgehalt bei jedem Entgasungs-Szenarium ermittelt wird,
- insbesondere daraus der insgesamt minimalste erreichbare Gasgehalt ermittelt oder abgeschätzt wird,
insbesondere
nach Ermittlung des Entgasungs-Verhaltens des Materials
- die optimalen Entgasungs-Parameter, etwa der Umgebungsdruck beim Entgasen, und/oder Betriebs-Parameter des Verbrauchers, etwa der Durchsatz des Verbrauchers, insbesondere automatisch, ermittelt werden,
- insbesondere in Abhängigkeit von vorgegebenen, vorzugsweise gestaffelt vorgegebenen, Optimierungszielen, etwa dem Energieverbrauch beim Aufbereiten des Materials (M),
- insbesondere der Gas-Gehalt während der Entgasung gemessen wird, insbesondere in zeitlichen Abständen gemessen wird.

18. Verfahren nach einem der Ansprüche 8 bis 17, **dadurch gekennzeichnet, dass**
bei der Messung des
- Gasgehalts an zwei in Strömungsrichtung des Materials (M) beabstandeten Stellen
- entweder die dabei gemessenen Gasgehalte als redundante Messwerte betrachtet und hieraus auch die korrekte Funktion der Sensoren geschlossen wird
- und/oder aus den dabei gemessenen Gasgehalten auf Eigenschaften der Aufbereitungs-Vorrichtung dazwischen, insbesondere auf Undichtigkeiten dazwischen, geschlossen wird.

## Claims

1. **Dispensing device** (100) for dispensing a liquid or pasty material (M), comprising the following modules:
- a consumer (60),
- a processing unit (50) with a processing container (51) for processing the material (M) and
- a conveyor unit (40, 40') for conveying the material (M)
wherein
the dispensing device (100) has at least one gas sensor (1) for determining the concentration of the measuring gas (G) in the material (M),
**characterised in that**
the gas sensor (1) is arranged in at least two positions spaced apart in the flow direction of the material (M) which are selected from the following:
- in the processing unit (50), stationary as position (F) or on an agitator blade (58b) as position (G);
- in a circulation pipe (52) on the processing unit (50) as position (D),
- in or on the consumer (60) as position (A),
- on or in the conveyor unit (40, 40') as position (C),
- in connecting pipes (70) between them as position (B).

2. Dispensing device according to claim 1,
**characterised in that**
the gas sensor (1) is an optical fluorescence quenching sensor that measures oxygen in a gas mixture
and/or
- the dispensing device (100) serves to dispense a liquid or pasty, self-levelling material (M) and the consumer (60) is a dispensing unit (60) for dispensing the material (60),
and/or
- the processing unit (50) is designed with a processing container (51) for processing the material (M) according to the requirements of the consumer (60), in which in particular the material is in contact with a known, defined gas or gas mixture,
and/or
- the conveyor unit (40, 40') is designed to convey the material (M) from a storage container (30) to the consumer (60),
wherein the
gas sensor (1) is designed to determine the concentration of the measuring gas (G), which is a measuring component of a gas mixture to be measured, in the material (M).

3. Dispensing device according to any of the preceding claims,
**characterised in that**
- the gas sensor (1) is an oxygen-measuring gas sensor (1)
and/or
- the gas sensor (1) is a sensor (1) that is in contact with the material
and/or
- the gas sensor (1) is a sensor (1) which consumes the measuring gas, in particular the measuring component of a gas mixture to be measured
and/or
- the gas sensor (1) is an optical sensor (1.1) that measures the oxygen concentration in the material (M).

4. Dispensing device according to claim 3,
**characterised in that**
the optical sensor (1.1) has an optically active layer between the radiation source and the material which is excited by the incident radiation and emits (secondary) radiation, in particular of a defined emission spectrum, which is detected by a suitable sensor, the presence of the measuring gas in the material changing, in particular reducing, the intensity and/or emission spectrum of the light emitted by the excitation.

5. Dispensing device according to any of the preceding claims,
**characterised in that**
- the gas sensor (1) is an amperometric sensor (1.2),
- in particular, the gas sensor (1.2) is a Clark sensor (1.2) that measures the oxygen concentration in a liquid,
- in particular, the sensor elements (1a, b) on the amperometric sensor (1.2), in particular on the Clark sensor, are covered by a membrane (3) that is permeable to the measuring gas.

6. Dispensing device according to any of the preceding claims,
**characterised in that**
in particular in the case of a sensor (1) which consumes the measuring gas
- the sensor (1) is arranged at a position in the dispensing device at which the material (M) flows along the contact surface of the sensor (1) on which the concentration of measuring gas is measured or through which measuring gas enters the sensor, at least during the operation of the dispensing device,
- in particular with the contact surface positioned at an angle to the flow direction, so that the flow passes against the contact surface,
- in particular in a pipeline or hose line for the material (M).

7. Dispensing device according to any of the preceding claims,
**characterised in that**
- a movement device (56), in particular an agitator (56), is present in the processing container,
- the movement device (56) is capable of maintaining a sufficiently strong flow of the material (M),
- in particular at the boundary layer facing the gas sensor, so that the subsequent delivery of the measuring gas, in particular of the measuring component, to the contact surface of the sensor (1.2) is greater than the consumption of the measuring gas by the amperometric gas sensor (1.2),
- in particular the upper region of the interior of the processing container (51) and/or the storage container (30) can be connected to a vacuum source (59),
- the vacuum source (59) is able to keep the gas mixture in the headspace of the processing container above the material at a pressure below 900 mbar, preferably below 500 mbar, preferably below 100 mbar, preferably below 50 mbar, preferably below 20 mbar, preferably below 10 mbar, preferably below 5 mbar.

8. **Method** for determining the gas concentration in a liquid or pasty material (M), wherein
- the concentration of such a measuring gas in the material (M) is measured,
- of which it is known that the material (M) was previously in contact with it and, in the case of a gas mixture, whose composition is known,
- in the case of a gas mixture, the concentration of the gas mixture as a whole in the material (M) is deduced from the concentration of this measuring component,
wherein
- the material is processed according to the requirements of a consumer (60) and is then consumed by a consumer (60) at a consumption location away from the processing location,
**characterised in that**
- the concentration of the measuring gas is measured in at least two positions spaced apart in the flow direction of the material (M), which are selected from:
- a position (A) where the concentration is measured as close as possible to the consumer (60),
- a position (F, G) where the concentration is measured at the processing location,
- a position (D) where the concentration in a circulation pipe (70) is measured at the processing location,
- a position (C) where the concentration is measured in a conveyor unit (40) conveying the material (M),
- a position (B) in connecting pipes (70) located between them.

9. Method according to claim 8,
**characterised in that**
the method is carried out in the context of a dispensing device (100) according to any of the preceding device claims,
and/or
the gas mixture contains oxygen and an optical fluorescence quenching measurement method is used,
and/or
the gas mixture used is one that contains oxygen, in particular air,
- an optical measuring method is used,
- in particular fluorescence quenching
and/or
- an amperometric measuring method is used,
- so that in particular the concentration of dissolved oxygen is also measured.

10. Method according to one of claims 8 to 9,
wherein a sensor principle is applied whereby the measuring gas, whose concentration is measured, is at least partially consumed by the sensor (1), in particular an amperometric sensor (1.2),
**characterised in that**
- a measurement is carried out if it can be assumed that, in the boundary layer of the material (M) at the sensor (1) and/or the sensor element of the sensor (1), the same concentration of measuring gas is present as exists on average in the rest of the material (M) within the container or module in which the measurement is carried out,
- or the concentration is representative of the concentration of measuring gas in the rest of the material (M) within the container or module being measured,
- in particular, is representative of the average concentration present in the material as a whole.

11. Method according to any of claims 8 to 10,
**characterised in that**
- the gas mixture is kept at a negative pressure of less than 900 mbar, preferably less than 500 mbar, preferably less than 100 mbar, preferably less than 50 mbar, preferably less than 20 mbar, preferably less than 10 mbar, preferably less than 5 mbar, and in particular the negative pressure is maintained in contact with the material (M)
and/or
- the concentration of measuring gas is measured by means of a gas sensor (1) in contact with the material (M), and
- the gas sensor (1) is arranged and used in such a way as to prevent the formation of a boundary layer of material (M) that moves very slowly or is even stationary relative to the sensor (1).

12. Method according to any of claims 8 to 11,
**characterised in that**
a sufficiently strong flow of the material (M), in particular at the boundary layer facing the gas sensor (1), is maintained during the measurement so that the subsequent delivery of the measuring gas to the contact surface of the sensor (1) taking place due to the flow is greater than the consumption of the measuring gas by the consuming gas sensor (1).

13. Method according to any of claims 8 to 12,
**characterised in that**
- the sensor elements of the gas sensor (1) are covered by a membrane (3) that is permeable to the measuring component of the gas,
- in particular the diffusivity of the membrane (3) for the measuring gas is known, so that the ratio of the concentration of measuring gas on the two sides of the membrane (3) is also known or can be determined, in particular with the aid of a calibration process.

14. Method according to any of claims 8 to 13,
wherein the method serves to determine the gas concentration in a liquid or pasty, self-levelling material (M),
- in particular whether a specified maximum permitted gas concentration in the material (M) is reached or fallen below, especially during its degassing,
wherein
- the concentration of a measuring component of the gas mixture is measured in the material (M),
- of which it is known that the material (M) was previously in contact with it and, in the case of a gas mixture, whose composition is known,
- in the case of a gas mixture, the concentration of the gas mixture in the material (M) as a whole is extrapolated from the concentration of this measuring component,

15. Method according to any of claims 8 to 14,
**characterised in that**
- the material (M) is only classified as usable, in particular automatically, with regard to the gas concentration, in particular material is only supplied to the consumer (60), when the gas concentration is below a permitted upper limit value,
- in particular if the processing is carried out in a processing container (51) at the processing location,
- the processing container (51) is refilled with material (M) in batches or continuously from a storage container (30), removal from the processing container (51) for the consumer (60) in particular automatically, only being permitted if the material (M) is classified as usable with regard to the gas concentration.

16. Method according to any of claims 8 to 15,
**characterised in that**
material (M) is only classified as usable with regard to the gas concentration if
- the gas concentration remained below a specified upper limit value for a specified continuity period
and/or
- the decrease in the gas concentration during a decrease period was below a specified decrease limit value
in particular:
in the case of abrasive material
- the sensor (1) is only exposed to, in particular flowing, material (M) during the measurement periods,
- the material bypasses the sensor (1), in particular outside the measurement periods.

17. Method according to any of claims 8 to 16,
**characterised in that**
in the case of a new material, in order to find out the degassing behaviour of the new material,
- new material is degassed from the same initial state over a degassing period and the gas concentration is measured while different degassing scenarios are present in the form of parameters influencing the degassing, in particular the ambient pressure and/or the material temperature during degassing and/or the amount of energy introduced to move the material during degassing,
- in particular, the minimum achievable gas concentration is determined for each degassing scenario,
- in particular, the overall minimum achievable gas concentration is determined or estimated,
in particular:
after determining the degassing behaviour of the material
- the optimum degassing parameters such as the ambient pressure during degassing and/or operating parameters of the consumer, such as the throughput of the consumer, are determined in particular automatically,
- in particular as a function of specified, preferably staggered, optimisation targets, such as the energy consumption when processing the material (M),
- in particular, the gas concentration is measured during degassing, in particular at time intervals.

18. Method according to any of claims 8 to 17,
**characterised in that**
when measuring the
- gas concentration at two points spaced apart in the flow direction of the material (M)
- either the measured gas concentrations are regarded as redundant measured values and the correct functioning of the sensors is also deduced therefrom
- and/or characteristics of the processing device between the points, in particular leakages between the points, are deduced from the measured gas concentrations.

## Revendications

1. Dispositif d'épandage (100) pour épandre un matériau (M) liquide ou pâteux, comprenant les modules suivants :
- un consommateur (60),
- une unité de préparation (50) ayant un récipient de préparation (51) pour préparer le matériau (M), et
- une unité de transport (40, 40') pour transporter le matériau (M),
dans lequel
le dispositif d'épandage (100) comprend au moins un capteur de gaz (1) pour déterminer la teneur en gaz de mesure (G) dans le matériau (M),
**caractérisé en ce que**
le capteur de gaz (1) est placé à au moins deux positions espacées dans la direction d'écoulement du matériau (M), qui sont choisies parmi :
- une position fixe (F) dans l'unité de préparation (50), ou une position (G) sur une pale d'agitation (58b),
- une position (D) dans une conduite de circulation (52) sur l'unité de préparation (50),
- une position (A) dans ou sur le consommateur (60),
- une position (C) sur ou dans l'unité de transport (40, 40'),
- une position (B) dans des conduites de liaison (70) interposées.

2. Dispositif d'épandage selon la revendication 1,
**caractérisé en ce que**
le capteur de gaz (1) est un capteur optique de quenching de fluorescence mesurant l'oxygène dans un mélange gazeux, et/ou
- le dispositif d'épandage (100) sert à épandre un matériau (M) liquide ou pâteux, auto-nivelant, et le consommateur (60) est une unité d'épandage (60) pour épandre le matériau (60),
et/ou
- l'unité de préparation (50) ayant un récipient de préparation (51) est conçue pour préparer le matériau (M) selon les exigences du consommateur (60), dans lequel, en particulier, le matériau est en contact avec un gaz ou un mélange gazeux connu et défini,
et/ou
- l'unité de transport (40, 40') est conçue pour transporter le matériau (M) d'un réservoir de stockage (30) vers le consommateur (60),
le capteur de gaz (1) est conçu pour déterminer la teneur en gaz de mesure (G) d'un composant de mesure à mesurer d'un mélange gazeux dans le matériau (M).

3. Dispositif d'épandage selon l'une des revendications précédentes,
**caractérisé en ce que**
- le capteur de gaz (1) est un capteur de gaz mesurant l'oxygène,
et/ou
- le capteur de gaz (1) est un capteur (1) venant en contact avec le matériau, et/ou
- le capteur de gaz (1) est un capteur (1) qui consomme le gaz de mesure, en particulier le composant de mesure à mesurer d'un mélange gazeux, et/ou
- le capteur de gaz (1) est un capteur optique (1.1) mesurant la teneur en oxygène dans le matériau (M).

4. Dispositif d'épandage selon la revendication 3,
**caractérisé en ce que**
le capteur optique (1.1) comporte une couche optiquement active entre la source de rayonnement et le matériau, qui est excitée par le rayonnement incident et émet un rayonnement (secondaire), en particulier d'un spectre d'émission défini, qui est détecté par un capteur approprié, et la présence du gaz de mesure dans le matériau modifie, en particulier réduit, l'intensité et/ou le spectre d'émission de la lumière émise par l'excitation.

5. Dispositif d'épandage selon l'une des revendications précédentes,
**caractérisé en ce que**
- le capteur de gaz (1) est un capteur ampérométrique (1.2),
- en particulier, le capteur de gaz (1.2) est un capteur de Clark (1.2) mesurant la teneur en oxygène dans un liquide,
- en particulier, les éléments de détection (1a, b) sur le capteur ampérométrique (1.2), en particulier sur le capteur de Clark, sont recouverts par une membrane (3) qui est perméable au gaz de mesure.

6. Dispositif d'épandage selon l'une des revendications précédentes,
**caractérisé en ce que**
en particulier dans le cas d'un capteur (1) qui consomme le gaz de mesure
- celui-ci est placé dans le dispositif d'épandage à une position telle qu'il existe un écoulement du matériau (M) le long de la surface de contact du capteur (1) avec le matériau, où la teneur en gaz de mesure est mesurée ou par laquelle le gaz de mesure pénètre dans le capteur, au moins pendant le fonctionnement du dispositif d'épandage,
- en particulier avec une inclinaison de la surface de contact par rapport à la direction d'écoulement de telle sorte que la surface de contact soit balayée par l'écoulement,
- en particulier dans une conduite tubulaire ou dans un tuyau flexible pour le matériau (M).

7. Dispositif d'épandage selon l'une des revendications précédentes,
**caractérisé en ce que**
- un dispositif de mise en mouvement (56), en particulier un agitateur (56), est présent dans le récipient de préparation,
- le dispositif de mise en mouvement (56) est en mesure d'assurer un écoulement du matériau (M),
- en particulier au niveau de la couche limite vers le capteur de gaz, ledit écoulement étant aussi important que la fourniture ultérieure du gaz de mesure, en particulier du composant de mesure, à la surface de contact du capteur (1.2), qui a lieu grâce à l'écoulement, soit supérieure à la consommation du gaz de mesure par le capteur de gaz ampérométrique (1.2),
- en particulier la zone supérieure de l'espace intérieur du récipient de préparation (51) et/ou du réservoir de stockage (30) peut être mise en communication avec une source de dépression (59),
- la source de dépression (59) est en mesure de maintenir le mélange gazeux, dans l'espace à gaz du récipient de préparation au-dessus du matériau, à une pression inférieure à 900 mbar, mieux inférieure à 500 mbar, mieux inférieure à 100 mbar, mieux inférieure à 50 mbar, mieux inférieure à 20 mbar, mieux inférieure à 10 mbar, mieux inférieure à 5 mbar.

8. Procédé de détermination de la teneur en gaz dans un matériau (M) liquide ou pâteux,
dans lequel
- la teneur en un tel gaz de mesure est mesurée dans le matériau (M),
- dont on sait que le matériau (M) a été préalablement en contact avec lui et dont on connaît, dans le cas d'un mélange gazeux, la composition,
- dans le cas d'un mélange gazeux, on déduit de la teneur en ce composant de mesure la teneur en mélange gazeux dans son ensemble dans le matériau (M),
sachant que
- le matériau est préparé selon les exigences d'un consommateur (60) et est alors consommé par le consommateur (60) sur un site de consommation à l'écart du site de préparation,
**caractérisé en ce que**
- la teneur en gaz de mesure est mesurée à au moins deux positions espacées dans la direction d'écoulement du matériau (M), qui sont choisies parmi :
- une position (A) où la teneur est mesurée aussi près que possible du consommateur (60),
- une position (F, G) où la teneur est mesurée sur le site de préparation,
- une position (D) où la teneur est mesurée dans une conduite de circulation (70) sur le site de préparation,
- une position (C) où la teneur est mesurée dans une unité de transport (40) transportant le matériau (M),
- une position (B) dans des conduites de communication (70) interposées.

9. Procédé selon la revendication 8,
**caractérisé en ce que**
le procédé est mis en oeuvre dans le cadre d'un dispositif d'épandage (100) selon l'une des revendications précédentes du dispositif,
et/ou
le mélange gazeux contient de l'oxygène et on utilise un procédé de mesure optique par quenching de fluorescence,
et/ou
on utilise comme mélange gazeux un mélange qui contient de l'oxygène, en particulier de l'air,
- on utilise un procédé de mesure optique,
- en particulier le quenching de fluorescence
et/ou
- on utilise un procédé de mesure ampérométrique,
- afin de mesurer en particulier aussi la teneur en oxygène dissous.

10. Procédé selon l'une des revendications 8 à 9,
dans lequel on utilise un principe de capteur dans lequel le gaz de mesure, dont la teneur est mesurée, est consommé au moins partiellement par le capteur (1), en particulier un capteur ampérométrique (1.2),
**caractérisé en ce que**
- on réalise une mesure lorsqu'on peut partir du principe que la même teneur en gaz de mesure est présente dans la couche limite du matériau (M) au niveau du capteur (1) et/ou de l'élément de détection du capteur (1) qu'en moyenne dans le reste du matériau (M) à l'intérieur du récipient ou du module dans lequel on réalise la mesure,
- ou une teneur qui est représentative de la teneur en gaz de mesure dans le reste du matériau (M) à l'intérieur du récipient ou du module dans lequel on réalise la mesure,
- en particulier représentative de la teneur moyenne présente dans l'ensemble du matériau.

11. Procédé selon l'une des revendications 8 à 10,
**caractérisé en ce que**
- le mélange gazeux est maintenu à une dépression inférieure à 900 mbar, mieux inférieure à 500 mbar, mieux inférieure à 100 mbar, mieux inférieure à 50 mbar, mieux inférieure à 20 mbar, mieux inférieure à 10 mbar, mieux inférieure à 5 mbar, et en particulier la dépression est maintenue en contact avec le matériau (M),
et/ou
- la mesure de la teneur en gaz de mesure est effectuée au moyen d'un capteur de gaz (1) en contact avec le matériau (M), et
- le capteur de gaz (1) est placé et utilisé de manière à empêcher la formation d'une couche limite de matériau (M) se déplaçant très lentement ou même immobile par rapport au capteur (1).

12. Procédé selon l'une des revendications 8 à 11,
**caractérisé en ce que**
un écoulement du matériau (M), en particulier sur la couche limite vers le capteur de gaz (1), est maintenu pendant la mesure à un niveau tel que la fourniture ultérieure du gaz de mesure à la surface de contact du capteur (1), qui a lieu grâce à l'écoulement, est supérieure à la consommation du gaz de mesure par le capteur de gaz (1) qui le consomme.

13. Procédé selon l'une des revendications 8 à 12,
**caractérisé en ce que**
- les éléments de détection du capteur de gaz (1) sont recouverts d'une membrane (3) qui est perméable au composant de mesure du gaz,
- en particulier la capacité de diffusion de la membrane (3) pour le gaz de mesure est connue, de sorte que le rapport de la teneur en gaz de mesure de part et d'autre de la membrane (3) est également connu ou peut être déterminé, en particulier à l'aide d'une opération de calibrage.

14. Procédé selon l'une des revendications 8 à 13,
dans lequel le procédé sert à déterminer la teneur en gaz dans un matériau (M) liquide ou pâteux auto-nivelant,
- en particulier, lorsqu'on atteint ou passe en-dessous d'une teneur de gaz prédéfinie maximale admissible du matériau (M), en particulier pendant son dégazage,
sachant que
- la teneur en un composant de mesure du mélange gazeux est mesurée dans le matériau (M),
- dont on sait que le matériau (M) a été préalablement en contact avec lui et dont on connaît, dans le cas d'un mélange gazeux, la composition,
- dans le cas d'un mélange gazeux, on déduit de la teneur en ce composant de mesure la teneur en mélange gazeux dans son ensemble dans le matériau (M).

15. Procédé selon l'une des revendications 8 à 14,
**caractérisé en ce que**
- le matériau (M) n'est classé comme utilisable, en particulier automatiquement, en ce qui concerne la teneur en gaz, en particulier le matériau n'est amené au consommateur (60) que lorsque la teneur en gaz est inférieure à une valeur limite supérieure admissible,
- en particulier lorsque, sur le site de préparation, la préparation est effectuée dans un récipient de préparation (51),
- le récipient de préparation (51) est rempli par lots ou en continu de matériau (M) à partir d'un réservoir de stockage (30), un prélèvement dans le récipient de préparation (51) n'est autorisé pour le consommateur (60), en particulier automatiquement, que lorsque le matériau (M) est classé comme utilisable en ce qui concerne la teneur en gaz.

16. Procédé selon l'une des revendications 8 à 15,
**caractérisé en ce que**
le matériau (M) n'est classé comme utilisable en ce qui concerne la teneur en gaz que lorsque
- la teneur en gaz est restée inférieure à une valeur limite supérieure prédéfinie pendant une période de continuité prédéfinie,
et/ou
- la diminution de la teneur en gaz pendant une période de diminution était inférieure à une valeur limite de diminution prédéfinie,
en particulier
dans le cas d'un matériau abrasif
- le capteur (1) n'est exposé au matériau (M), en particulier en écoulement, que pendant les temps de mesure,
- en particulier en dehors des temps de mesure, le matériau est guidé de manière à passer devant le capteur (1).

17. Procédé selon l'une des revendications 8 à 16,
**caractérisé en ce que**
dans le cas d'un nouveau matériau, pour apprendre à connaître le comportement de dégazage du nouveau matériau
- un nouveau matériau est dégazé à partir du même état initial pendant une durée de dégazage, et la teneur en gaz est mesurée en présence de différents scénarios de dégazage sous forme de paramètres influençant le dégazage, en particulier la pression ambiante et/ou la température du matériau lors du dégazage et/ou la quantité d'énergie apportée pour la mise en mouvement du matériau pendant le dégazage,
- en particulier, la teneur minimale en gaz pouvant être atteinte pour chaque scénario de dégazage est déterminée,
- en particulier, la teneur totale minimale en gaz pouvant être atteinte est déterminée ou estimée,
en particulier
après avoir déterminé le comportement de dégazage du matériau
- les paramètres de dégazage optimaux, par exemple la pression ambiante lors du dégazage, et/ou les paramètres de fonctionnement du consommateur, par exemple le débit du consommateur, sont déterminés, en particulier automatiquement,
- en particulier en fonction d'objectifs d'optimisation prédéfinis, de préférence échelonnés, tels que la consommation d'énergie lors de la préparation du matériau (M),
- en particulier la teneur en gaz est mesurée pendant le dégazage, en particulier à intervalles de temps.

18. Procédé selon l'une des revendications 8 à 17,
**caractérisé en ce que**
lors de la mesure
- de la teneur en gaz à deux endroits espacés dans la direction d'écoulement du matériau (M),
- on considère les teneurs en gaz ainsi mesurées comme des valeurs de mesure redondantes et on en déduit également le fonctionnement correct des capteurs,
- et/ou on déduit des teneurs en gaz mesurées à cette occasion les propriétés du dispositif de préparation entre les deux endroits, en particulier les défauts d'étanchéité entre les deux endroits.
